# EUROPÄISCHE PATENTSCHRIFT

(11) **EP 0 888 261 B1**
(45) Veröffentlichungstag und Bekanntmachung des Hinweises auf die Patenterteilung: **28.11.2001**
(21) Anmeldenummer: 97907075.2
(22) Anmeldetag: 06.03.1997
(51) Int. Cl.: C07B 37/04, C07C 205/12, C07C 205/06, C07C 205/35, C07C 205/11, C07C 201/12

(54) **VERFAHREN ZUR HERSTELLUNG VON NITROBIPHENYLEN**
PROCESS FOR PREPARING NITROBIPHENYLENE
PROCEDE DE PREPARATION DE NITROBIPHENYLENE

(30) Priorität: 13.03.1996 DE 19609765
(43) Veröffentlichungstag der Anmeldung: 07.01.1999
(73) Patentinhaber: BASF AKTIENGESELLSCHAFT, 67056 Ludwigshafen (DE)
(72) Erfinder: EICKEN, Karl, D-67157 Wachenheim (DE); GEBHARDT, Joachim, D-67157 Wachenheim (DE); RANG, Harald, 43007 Tarragona (ES); RACK, Michael, D-69123 Heidelberg (DE); SCHÄFER, Peter, D-67308 Ottersheim (DE)
(74) Vertreter: Riedl, Peter, Dr.
(86) Internationale Anmeldenummer: EP9701128
(87) Internationale Veröffentlichungsnummer: WO9733846

(56) Entgegenhaltungen:
- EP-A- 0 606 065
- TETRAHEDRON LETTERS, Bd. 37, Nr. 26, 24.Juni 1996, Seiten 4499-4502, XP000591551 REETZ M T ET AL: "SUZUKI AND HECK REACTIONS CATALYZED BY PREFORMED PALLADIUM CLUSTERS AND PALLADIUM/NICKEL BIMETALLIC CLUSTERS"
- SYNTHESIS, 1989, STUTTGART DE, Seiten 184-188, XP002030760 T. IIHAMA ET AL.: "REGIOSPECIFIC SYNTHESES OF ALL ISOMERIC NITROFLUORENONES AND NITROFLUORENES BY TRANSITION METAL CATALYZED CROSS-COUPLING REACTIONS"
- JOURNAL OF ORGANIC CHEMISTRY, Bd. 59, 1994, EASTON US, Seiten 5034-5037, XP002030761 T.I. WALLOW ET AL.: "HIGHLY EFFICIENT AND ACCELERATED SUZUKI ARYL COUPLINGS MEDIATED BY PHOSPHINE-FREE PALLADIUM SOURCES"
- ANGEWANDTE CHEMIE INTERNATIONAL EDITION., Bd. 34, Nr. 17, 1995, WEINHEIM DE, Seiten 1848-1849, XP002030762 M. BELLER ET AL.: "PALLADACYCLES AS EFFICIENT CATALYSTS FOR ARYL COUPLING REACTIONS"

## Beschreibung

Die vorliegende Erfindung betrifft ein Verfahren zur Herstellung von Nitrobiphenylen der Formel I 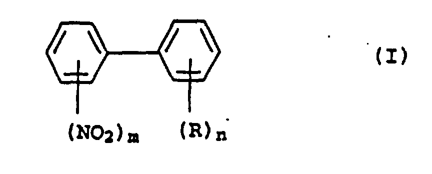 in der m für 1 oder 2 steht, R Halogen, R' oder OR' bedeutet, wobei R' ein C-organischer Rest ist, welcher unter den Reaktionsbedingungen inerte Gruppen tragen kann, wobei n für 0, 1, 2 oder 3 steht und im Falle, daß n 2 oder 3 ist, die Reste R auch verschieden sein können, welches dadurch gekennzeichnet ist, daß man ein Chlornitrobenzol der Formel II 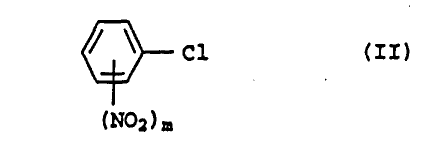 in Gegenwart einer Base und eines Palladium-Katalysators ausgewählt aus der Gruppe: a) Palladium-Triarylphosphankomplex mit Palladium in der Oxydationsstufe Null, b) Salz des Palladiums in Gegenwart von Triarylphosphan als Komplexligand oder c) gegebenenfalls auf Träger aufgezogenes metallisches Palladium, in Gegenwart von Triarylphosphan in einem Lösungsmittel mit einer Phenylboronsäure (IIIa) 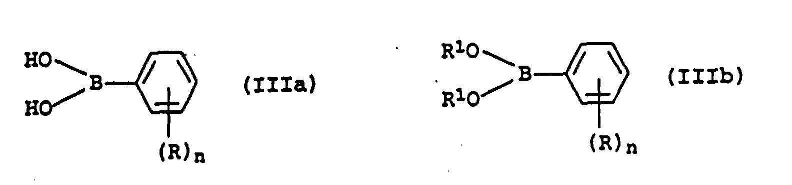 einem ihrer Alkylester der Formel IIIb, wobei R¹ die Bedeutung C₁-C₆-Alkyl hat, oder einem ihrer Anhydride umsetzt.

Beller et al. (Angew. Chem. Int. Ed. Bd.34, 1995, 1848-1849) beschreiben die Arylkupplung von Phenylboronsäure mit 4-Chloracetophenon in Gegenwart von Palladacyclen in denen 2 Palladiumatome, die jeweils Bestandteil eines Benzodihydrophospholrings sind, über zwei Acetatgruppen zu einem Bis-Palladium-Cyclus verbrückt sind. Eine analoge Umsetzung in Gegenwart von Palladiumacetat und Triphenylphosphin liefert kein Kupplungsprodukt.

Wallow et al. (J. Org. Chem. Bd. 59, 1994, S. 5034-5037) beschreiben die Suzuki-Kupplung von 4-Iod- und 4-Bromnitrobenzol mit Phenylboronsäureestern in Gegenwart phosphinfreier Palladium-katalysatoren.

Iihama et al. (Synthesis, 1989, 184-188) beschreiben die regiospezifische Herstellung von Nitrofluorenonen durch Übergangsmetall-katalysierte Kreuzkupplungsreaktionen. Zur Kreuzkupplung werden Phenylboronsäuren mit Nitrobrombenzol-Derivaten in Gegenwart von Tetrakistriphenylphosphan-Palladium umgesetzt.

Aus Synth. Commun. 11, Seite 513 (1981) ist bekannt, daß Phenylboronsäure mit Chlorbenzol in Gegenwart von Tetrakis(triphenylphosphan)palladium und Natriumethanolat nicht zu Biphenyl gekuppelt werden kann.

Tetrahedron Lett. 32, Seite 2277 (1991) ist zu entnehmen, daß die Kupplungsreaktion zwischen Phenylboronsäure und Chlorbenzol bei Verwendung des Katalysators [1,4-bis-(Diphenylphosphan)butan]palladium (II) dichlorid mit einer Ausbeute von lediglich 28 % verläuft.

Der vorliegenden Erfindung lag daher die Aufgabe zugrunde, ein wirtschaftliches Verfahren zur Herstellung von Nitrobiphenylen bereitzustellen, welches mit gut zugänglichen Palladium-Katalysatoren arbeitet.

Demgemäß wurde das eingangs definierte Verfahren gefunden.

Die Phenylboronsäuren IIIa, deren Ester IIIb und Anhydride wie IIIc, welche im folgenden zusammenfassend als "Borverbindungen III" bezeichnet werden, sind allgemein bekannt oder in an sich bekannter Weise erhältlich (vgl. z.B. Org. Synth. Coll. Vol. IV, Seite 68).

Bevorzugte C-organische Reste R' sind:
- Alkyl- und Alkenylgruppen, insbesondere mit 1 bis 12 Kohlenstoffatomen wie Methyl, Ethyl, Propyl, Butyl und Allyl,
- Alkylcarbonyl- und Alkoxycarbonylgruppen, insbesondere mit 1 bis 6 Kohlenstoffatomen wie Acetyl, Methoxycarbonyl und Ethoxycarbonyl,
- Cycloalkylgruppen, insbesondere mit 3 bis 10 Kohlenstoffatomen wie Cyclopentyl, Cyclohexyl und 1-Methylcyclohexyl sowie
- Phenyl- und Phenoxygruppen.

Unter den Reaktionsbedingungen inerte Substituenten des C-organischen Restes R' sind vorzugsweise Halogen und daneben Alkyl- und Alkoxygruppen.

Weitere C-organische Reste R' sind:
- Cyano- und die Formylgruppen (-CHO)

In den Anhydriden sind normalerweise Produkte der Zusammenlagerung von zwei oder mehreren Äquivalenten Phenylboronsäure IIIa unter Wasseraustritt, welche intermolekulare B-O-B-Brücken enthalten. Bevorzugt sind cyclische Anhydride der Formel IIIc.

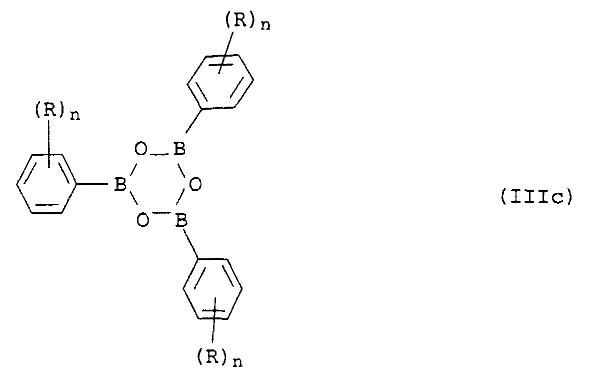

Bei den Stoffmengenangaben, welche sich auf die Borverbindungen III beziehen, ist das im folgenden zu berücksichtigen. Diese Mengenangaben beziehen sich stets auf Phenylborsäure-Äquivalente.

Im allgemeinen können Alkylester der Formel IIIb, in der R¹ die Bedeutung C₁-C₆-Alkyl hat, eingesetzt werden. Bevorzugte Alkylester IIIb sind die Dimethylester und die Diethylester.

Vorzugsweise geht man bei dem erfindungsgemäßen Verfahren von den Phenylboronsäuren IIIa aus.

Weiterhin geht man bevorzugt von Borverbindungen der Formel III aus, in denen R für eine C₁-C₄-Alkylgruppe oder Halogen und insbesondere für Methyl, Fluor oder Chlor steht.

Darüberhinaus sind Borverbindungen III als Ausgangsmaterial bevorzugt, in denen n für 1 und insbesondere für 0 steht.

Ganz besonders bevorzugt sind 4-Methylphenylboronsäure, 4-Fluorphenylboronsäure und vor allem 4-Chlorphenylboronsäure als Ausgangsverbindung IIIa.

Vorzugsweise geht man von Nitrochlorbenzolen II aus, welche eine einzige Nitrogruppe tragen (m = 1), insbesondere 4-Nitrochlorbenzol und vor allem 2-Nitrochlorbenzol.

Die Borverbindungen III (Phenylboronsäure-Äquivalente) werden, bezogen auf die Verbindungen II, normalerweise mit bis zu 50-prozentigem, vorzugsweise mit bis zu 20-prozentigem Überschuß und besonders bevorzugt äquimolar eingesetzt.

Als Base können organische Basen wie z.B. tertiäre Amine eingesetzt werden. Bevorzugt verwendet man z.B. Triethylamin oder Dimethylcyclohexylamin.

Als Base verwendet man vorzugsweise Alkalimetallhydroxide, Erdalkalimetallhydroxide, Alkalimetallcarbonate, Erdalkalimetallcarbonate, Alkalimetallhydrogencarbonate, Alkalimetallacetate, Erdalkalimetallacetate, Alkalimetallalkoholate und Erdalkalimetallalkoholate, im Gemisch und insbesondere einzeln.

Als Base besonders bevorzugt sind Alkalimetallhydroxide, Erdalkalimetallhydroxide, Alkalimetallcarbonate, Erdalkalimetallcarbonate und Alkalimetallhydrogencarbonate.

Als Base ganz besonders bevorzugt sind Alkalimetallhydroxide, z.B. Natriumhydroxid und Kaliumhydroxid, sowie Alkalimetallcarbonate und Alkalimetallhydrogencarbonate, z.B. Lithiumcarbonat, Natriumcarbonat und Kaliumcarbonat.

Die Base wird im erfindungsgemäßen Verfahren vorzugsweise mit einem Anteil von 100 bis 500, besonders bevorzugt 150 bis 400 Mol-%, bezogen auf die Borverbindung III, eingesetzt.

Erfindungsgemäss werden als Palladium-Katalysatoren Palladium-Ligandenkomplexe mit Palladium in der Oxydationsstufe Null, Salze des Palladiums in Gegenwart von Komplexliganden oder gegebenenfalls auf Träger aufgezogenes metallisches Palladium in Gegenwart von Komplexliganden eingesetzt.

Geeignete Komplexliganden sind Triarylphosphane, welche in den Arylringen gegebenenfalls substituiert sein können. Die Wasserlöslichkeit der Palladium-Komplexe läßt sich durch folgenden Substituenten verbessern: Sulfonsäuresalzgruppen, Sulfonsäuregruppen, Carbonsäuresalzgruppen, Carbonsäuregruppen, Phosphonsäuresalzgruppen, Phosphonsäuregruppen, Phosphoniumgruppen, Peralkylammoniumgruppen, Hydroxygruppen und Polyethergruppen.

Aus den Palladium-Ligandenkomplexen mit Palladium in der Oxidationsstufe 0 verwendet man vorzugsweise Tetrakis(triphenylphosphan)palladium und daneben Tetrakis[tri(o-tolyl)phosphan]palladium.

In den Salzen des Palladiums, welche in Gegenwart von Komplexliganden verwendet werden, liegt das Palladium normalerweise in der zweifach positiven Oxydationsstufe vor. Bevorzugt verwendet man Palladiumacetat oder Palladiumchlorid.

In der Regel werden 2 bis 6 Äquivalente der vorstehend genannten Komplexliganden, insbesondere Triphenylphosphan, mit einem Äquivalent des Palladiumsalzes kombiniert (vgl. z.B. J. Org. Chem. 49, Seite 5240 (1984)).

Im übrigen sind derartige lösliche Palladium-Komplexe allgemein bekannt (vgl. z.B. Angew. Chem. 105, Seite 1589 (1993)), so daß sich weitere Ausführungen hierzu erübrigen.

Metallisches Palladium verwendet man vorzugsweise in pulverisierter Form oder auf einem Trägermaterial, z.B. als Palladium auf Aktivkohle, Palladium auf Aluminiumoxid, Palladium auf Bariumcarbonat, Palladium auf Bariumsulfat, Palladium auf Calciumcarbonat, Palladium auf Aluminiumsilikaten wie Montmorillonit, Palladium auf SiO₂ und Palladium auf Calciumcarbonat, jeweils mit einem Palladiumgehalt von 0,5 bis 12 Gew.-%. Diese Katalysatoren können neben Palladium und dem Trägermaterial weitere Dotierstoffe, z.B. Blei, enthalten.

Besonders bevorzugt ist bei der Verwendung von gegebenenfalls auf Träger aufgezogenem, metallischem Palladium die Mitverwendung der vorstehend genannten Komplexliganden, insbesondere die Verwendung von Palladium auf Aktivkohle in Gegenwart von Triphenylphosphan als Komplexligand, wobei die Phenylgruppen im Triphenylphosphan vorzugsweise mit insgesamt ein bis drei Sulfonatgruppen substituiert sind.

In der Regel werden 2 bis 3 Äquivalente der vorstehend genannten Komplexliganden pro Äquivalent des Palladiummetalls verwendet.

Der Palladium-Katalysator wird im erfindungsgemäßen Verfahren mit einem Anteil von 0,01 bis 10, vorzugsweise 0,05 bis 5 und insbesondere 0,1 bis 3 Mol-% bezogen auf die Verbindung II eingesetzt.

Das erfindungsgemäße Verfahren kann in einem Zweiphasensystem aus wäßriger Phase und fester Phase, d.h. dem Katalysator, durchgeführt werden. Die wäßrige Phase kann dabei neben Wasser auch ein wasserlösliches organisches Lösungsmittel enthalten.

Für das erfindungsgemäße Verfahren geeignete organische Lösungsmittel sind beispielsweise Ether, z.B. Dimethoxyethan, Diethylenglykoldimethylether, Tetrahydrofuran, Dioxan und tert.-Butylmethylether, Kohlenwasserstoffe, z.B. Hexan, Heptan, Cyclohexan, Benzol, Toluol und Xylol, Alkohole, z.B. Methanol, Ethanol, 1-Propanol, 2-Propanol, Ethylenglykol, 1-Butanol, 2-Butanol und tert.-Butanol, Ketone, z.B. Aceton, Ethylmethylketon und iso-Butylmethylketon, Amide, z.B. Dimethylformamid, Dimethylacetamid und N-Methylpyrrolidon, jeweils einzeln oder im Gemisch.

Bevorzugte Lösungsmittel sind Ether, z.B. Dimethoxyethan und Tetrahydrofuran, Kohlenwasserstoffe, z.B. Cyclohexan, Toluol und Xylol, Alkohole, z.B. Ethanol, 1-Propanol, 2-Propanol, 1-Butanol und tert.-Butanol, jeweils einzeln oder im Gemisch.

In einer besonders bevorzugten Variante werden im erfindungsgemäßen Verfahren Wasser, ein oder mehrere in Wasser unlösliche und ein oder mehrere in Wasser lösliche Lösungsmittel eingesetzt, beispielsweise Mischungen aus Wasser, Toluol und Ethanol oder Wasser, Toluol und Tetrahydrofuran, vorzugsweise jeweils im Volumenverhältnis 1:2:1.

Die Gesamtmenge an Lösungsmittel liegt normalerweise bei 3000 bis 500 und vorzugsweise bei 2000 bis 700 g pro Mol der Verbindung II.

Zweckmäßigerweise werden zur Durchführung des Verfahrens die Verbindung II, die Borverbindung III, die Base sowie die katalytische Menge des Palladium-Katalysators in eine Mischung aus Wasser und einem oder mehreren inerten organischen Lösungsmitteln gegeben und bei einer Temperatur von 0 bis 150, vorzugsweise 30 bis 120 °C für einen Zeitraum von 1 bis 50, vorzugsweise 2 bis 24 Stunden gerührt.

Die Durchführung kann in üblichen für derartige Verfahren geeigneten Apparaturen erfolgen, so daß sich weitere Ausführungen hierzu erübrigen.

Nach beendeter Umsetzung wird als Feststoff anfallender Palladium-Katalysator beispielsweise durch Filtration abgetrennt und das Rohprodukt vom Lösungsmittel bzw. den Lösungsmitteln befreit.

Bei nicht völlig wasserlöslichen Produkten werden wasserlösliche Palladium-Katalysatoren oder Komplexliganden bei der Trennung der Wasserphase vom Rohprodukt vollständig abgetrennt.

Anschließend kann nach dem Fachmann bekannten und dem jeweiligen Produkt angemessenen Methoden, z.B. durch Umkristallisation, Destillation, Sublimation, Zonenschmelzen, Schmelzkristallisation oder Chromatographie weiter aufgereinigt werden.

Bei Reaktionsende als Feststoff vorliegender Katalysator läßt sich in der Regel leicht abtrennen, regenerieren und wieder dem Prozeß zuführen, wodurch eine Senkung der Verfahrenskosten und eine Entlastung der Abfallprodukte von Palladium erreicht wird.

Mit dem erfindungsgemäßen Verfahren lassen sich beispielsweise herstellen:
4'-Fluor-2-nitrobiphenyl
4'-Methyl-2-nitrobiphenyl
4'-Methoxy-2-nitrobiphenyl
4'-Brom-2-nitrobiphenyl
3'-Fluor-2-nitrobiphenyl
3'-Chlor-2-nitrobiphenyl
3'-Brom-2-nitrobiphenyl
3'-Methyl-2-nitrobiphenyl
3'-Methoxy-2-nitrobiphenyl
4'-Phenyl-2-nitrobiphenyl
4'-Trifluormethyl-2-nitrobiphenyl
4'-Fluor-4-nitrobiphenyl
4'-Chlor-4-nitrobiphenyl
4'-Brom-4-nitrobiphenyl
4'-Methyl-4-nitrobiphenyl
4'-Cyano-4-nitrobiphenyl
2-Nitrobiphenyl
4-Nitrobiphenyl

Das erfindungsgemäße Verfahren liefert die Verbindungen I in hohen Ausbeuten bei sehr guter Reinheit.

Die mit dem erfindungsgemäßen Verfahren erhältlichen Nitrobiphenyle eignen sich als Vorprodukte für Biphenylamine, welche ihrerseits Zwischenprodukte sind für fungizide Pflanzenschutz-Wirkstoffe (vgl. EP-A 545 099).

Synthese von 4'-Chlor-2-nitrobiphenyl

### Beispiel 1

Zu einer Lösung von 9,45 g 2-Nitrochlorbenzol und 10,3 g 4-Chlorphenylboronsäure in 60 ml Tetrahydrofuran gab man eine Lösung von 9,6 g Natriumhydroxid in 60 ml Wasser unter Rühren und unter Stickstoffatmosphäre. Anschließend versetzte man die Mischung mit 70 mg Palladium(II)acetat und 370 mg Triphenylphosphan. Die Mischung wurde unter Rühren solange am Rückfluß gekocht (70 °C), bis das 2-Nitrochlorbenzol umgesetzt war (ca. 8 Stunden). Nach dem Abkühlen wurde der Ansatz mit 80 ml Wasser und 80 ml tert.-Butylmethylether versetzt und die organische Phase abgetrennt. Nach Filtration über 10 g Kieselgel und Verdampfen des Lösungsmittels erhielt man 13,95 g der Titelverbindung in einer Reinheit von 95 % (GC).

### Beispiel 2

Zu einer Lösung von 4,7 g 2-Nitrochlorbenzol und 5,6 g 4-Chlorphenylboronsäure in 30 ml 1,2-Dimethoxyethan gab man eine Lösung von 8 g Natriumcarbonat in 30 ml Wasser unter Rühren und unter Stickstoffatmosphäre. Anschließend versetzte man die Mischung mit 320 mg Palladium auf Aktivkohle (10 Gew.-%) und 320 mg Triphenylphosphan. Die Mischung wurde unter Rühren solange am Rückfluß gekocht, bis das 2-Nitrochlorbenzol umgesetzt war (ca. 22 Stunden). Nach dem Abkühlen wurde der Ansatz mit 50 ml Wasser und 50 ml tert.-Butylmethylether versetzt und die organische Phase abgetrennt. Nach Filtration und Verdampfen des Lösungsmittels erhielt man 6,7 g der Titelverbindung in einer Reinheit von 92,7 % (GC).

## Patentansprüche

1. Verfahren zur Herstellung von Nitrobiphenylen der Formel I 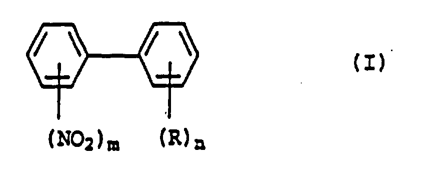 in der m für 1 oder 2 steht, R Halogen, R' oder OR' bedeutet, wobei R' ein C-organischer Rest ist, welcher unter den Reaktionsbedingungen inerte Gruppen tragen kann, wobei n für 0, 1, 2 oder 3 steht und im Falle, daß n 2 oder 3 ist, die Reste R auch verschieden sein können, **dadurch gekennzeichnet, daß** man ein Chlornitrobenzol der Formel II 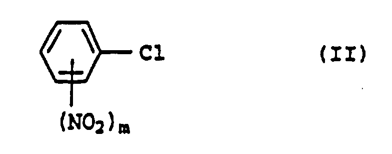 in Gegenwart einer Base und eines Palladium-Katalysators ausgewählt aus der Gruppe: a) Palladium-Triarylphosphankomplex mit Palladium in der Oxydationsstufe Null, b) Salz des Palladiums in Gegenwart von Triarylphosphan als Komplexligand oder c) gegebenenfalls auf Träger aufgezogenes metallisches Palladium, in Gegenwart von Triarylphosphan in einem Lösungsmittel mit einer Phenylboronsäure (IIIa) 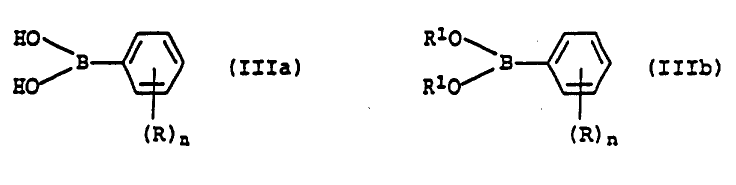 einem ihrer Alkylester der Formel IIIb, wobei R¹ die Bedeutung C₁-C₆-Alkyl hat, oder einem ihrer Anhydride umsetzt.

2. Verfahren nach Anspruch 1, **dadurch gekennzeichnet, daß** man als Verbindung II 2-Nitrochlorbenzol einsetzt.

3. Verfahren nach Anspruch 1, **dadurch gekennzeichnet, daß** man von einer Phenylboronsäure, welche nur in der 4-Position substituiert ist, als Verbindung IIIa und 2-Chlornitrobenzol als Verbindung II ausgeht.

4. Verfahren nach den Ansprüchen 1 bis 3, **dadurch gekennzeichnet, daß** man eine Phenylboronsäure IIIa einsetzt, welche als einzigen Substituenten in der 4-Position Fluor, Chlor oder eine Methylgruppe trägt.

5. Verfahren nach den Ansprüchen 1 bis 4, **dadurch gekennzeichnet, daß** man von 4-Chlorphenylboronsäure als Verbindung IIIa ausgeht.

6. Verfahren nach den Ansprüchen 1 bis 5, **dadurch gekennzeichnet, daß** man als Palladium-Katalysator a) gemäß Anspruch 1 Tetrakis (triphenylphosphan)palladium verwendet.

7. Verfahren nach den Ansprüchen 1 bis 5, **dadurch gekennzeichnet, daß** man einen Palladium-Katalysator b) gemäß Anspruch 1 verwendet.

8. Verfahren nach den Ansprüchen 1 bis 5, **dadurch gekennzeichnet, daß** man als Palladium-Katalysator c) gemäß Anspruch 1 metallisches Palladium auf Aktivkohle in Gegenwart von Triphenylphosphan verwendet, dessen Phenylgruppen mit insgesamt 1 bis 3 Sulfonatgruppen substituiert sind.

## Claims

1. A process for preparing nitrobiphenyls of the formula I 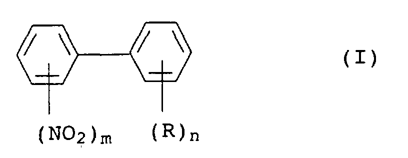 where m is 1 or 2, R is halogen, R' or OR', where R' is a C-organic radical which may carry groups inert under the reaction conditions, n is 0, 1, 2 or 3, and, in the case of n being 2 or 3, the radicals R are the same or different, which comprises reacting a chloronitrobenzene of the formula II 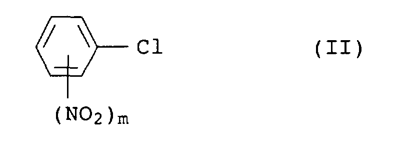 in the presence of a base and a palladium catalyst selected from the group consisting of a) palladium triarylphosphine complex having palladium in the oxidation state zero, b) palladium salt in the presence of triarylphosphine as a complexed ligand or c) metallic palladium which is, if appropriate, deposited on supports, in the presence of triarylphosphine in a solvent with a phenylboronic acid (IIIa) 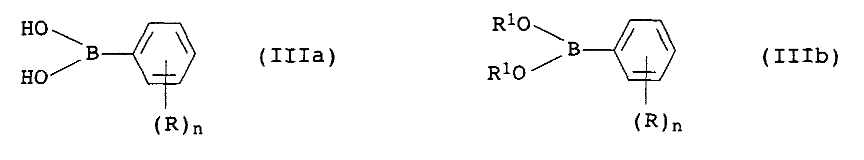 or an alkyl ester thereof of the formula IIIb, where R¹ is C₁-C₆-alkyl, or an anhydride thereof.

2. A process as claimed in claim 1, wherein the compound II used is 2-nitrochlorobenzene.

3. A process as claimed in claim 1, wherein the compound IIIa used is a phenylboronic acid which is only 4-substituted and the compound II used is 2-chloronitrobenzene.

4. A process as claimed in any of claims 1 to 3, wherein the phenylboronic acid IIIa used carries as only substituent in the 4 position fluorine, chlorine or a methyl group.

5. A process as claimed in any of claims 1 to 4, wherein the compound IIIa used is 4-chlorophenylboronic acid.

6. A process as claimed in any of claims 1 to 5, wherein the palladium catalyst a) used, which catalyst is claimed in claim 1, is tetrakis(triphenylphosphine)palladium.

7. A process as claimed in any of claims 1 to 5, wherein a palladium catalyst b) as claimed in claim 1 is used.

8. A process as claimed in any of claims 1 to 5, wherein the palladium catalyst c) used, which catalyst is claimed in claim 1, is metallic palladium on activated carbon in the presence of triphenylphosphine whose phenyl groups are substituted with a total of 1 to 3 sulfonate groups.

## Revendications

1. Procédé pour la préparation de nitrobiphénylène de formule I 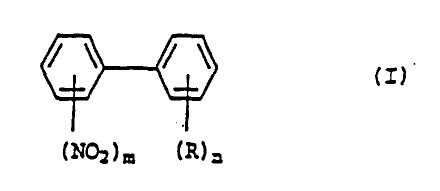 dans laquelle m vaut 1 ou 2, R désigne un halogène, R' ou OR', tandis que R' est un reste organique carboné qui peut porter des groupes inertes dans les conditions de réaction, et tandis que n vaut 0, 1, 2 ou 3, et dans le cas où n est 2 ou 3 les restes R peuvent également être différents, **caractérisé par le fait qu'**on fait réagir un chloronitrobenzène de formule II 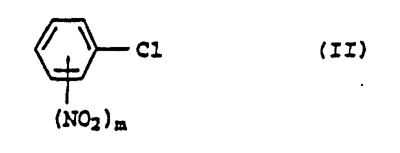 en présence d'une base et d'un catalyseur au palladium choisi dans le groupe: a) complexe triarylphosphane-palladium avec le palladium au stade d'oxydation zéro, b) sel du palladium en présence de triarylphosphane en tant que ligand du complexe ou c) palladium métallique éventuellement déposé sur support, en présence de triarylphosphane dans un solvant, avec un acide phénylborique (IIIa) 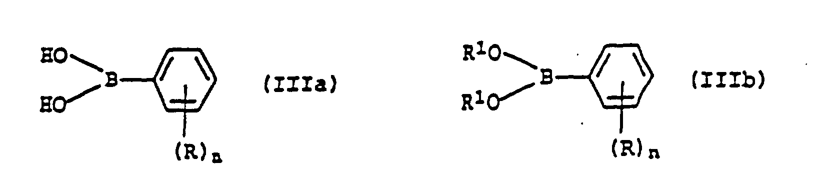 un de ses esters d'alkyle de formule IIIb, tandis que R¹ représente un alkyle en C₁-C₆, ou un de ses anhydrides.

2. Procédé selon la revendication 1, **caractérisé par le fait qu'**on emploie comme composé II du 2-nitrochlorobenzène.

3. Procédé selon la revendication 1, **caractérisé par le fait qu'**on part d'un acide phénylborique qui n'est substitué que sur la position 4, en tant que composé IIIa et de 2-chloronitrobenzène en tant que composé II.

4. Procédé selon les revendications 1 à 3, **caractérisé par le fait qu'**on utilise un acide phénylborique IIIa qui porte comme unique substituant sur la position 4 du fluor, du chlore ou un groupe méthyle.

5. Procédé selon les revendications 1 à 4, **caractérisé par le fait qu'**on part d'acide 4-chlorophénylborique en tant que composé IIIa.

6. Procédé selon les revendications 1 à 5, **caractérisé par le fait qu'**on utilise comme catalyseur au palladium a) selon la revendication 1 du tétrakis(triphénylphosphane)palladium.

7. Procédé selon les revendications 1 à 5,**caractérisé par le fait qu'**on utilise un catalyseur au palladium b) selon la revendication 1.

8. Procédé selon les revendications 1 à 5, **caractérisé par le fait qu'**on utilise comme catalyseur au palladium c) selon la revendication 1 du palladium métallique sur charbon actif en présence de triphénylphosphane, dont les groupes phényle sont substitués par au total 1 à 3 groupes sulfonate.
